Europäisches Patentamt

European Patent Office

Office européen des brevets

(11) Publication number: **0 370 694**
**A2**

## EUROPEAN PATENT APPLICATION

(21) Application number: 89311862.0

(22) Date of filing: 16.11.89

(51) Int. Cl.5: **C12Q 1/68, C12Q 1/70,**
**G01N 33/543**

(30) Priority: 21.11.88 US 273779

(43) Date of publication of application:
30.05.90 Bulletin 90/22

(84) Designated Contracting States:
AT BE CH DE FR GB IT LI LU NL SE

(71) Applicant: **EASTMAN KODAK COMPANY**
**343 State Street**
**Rochester, New York 14650(US)**

Applicant: **CETUS CORPORATION**
**1400 Fifty-Third Street**
**Emeryville California 94608(US)**

(72) Inventor: **Burdick, Brent Arthur c/o Eastman**
**Kodak Comp.**
**Patent Department 343 State Street**
**Rochester New York 14650(US)**
Inventor: **Oakes, Fred Terry Eastman Kodak**
**Comp.**
**Patent Department 343 State Street**
**Rochester New York 14650(US)**

Inventor: **Levenson, Corey Howard Eastman**
**Kodak Comp.**
**Patent Department 343 State Street**
**Rochester New York 14650(US)**
Inventor: **Fyles, Janet Linn Eastman Kodak**
**Comp.**
**Patent Department 343 State Street**
**Rochester New York 14650(US)**
Inventor: **Watson, Robert Malcolm Eastman**
**Kodak Comp.**
**Patent Department 343 State Street**
**Rochester New York 14650(US)**
Inventor: **Chang, Chu-An Eastman Kodak**
**Comp.**
**Patent Department 343 State Street**
**Rochester New York 14650(US)**

(74) Representative: **Nunney, Ronald Frederick**
**Adolphe et al**
**Kodak Limited Patent Department Headstone**
**Drive**
**Harrow Middlesex HA1 4TY(GB)**

(54) Diagnostic kit and method using a solid phase capture means for detecting nucleic acids.

(57) A method for the detection of a specific nucleic acid sequence of a predetermined nucleic acid in a specimen includes the use of oligonucleotide primers complementary to the sequence, a labeled probe and known amplification techniques to form primer extension products of the primers. At least one of the primers is labeled with a specific binding moiety which, at some point in the method, is complexed with a receptor thereto. The receptor is bound to a solid support material, such as polymeric particles. The complexation can occur either prior or subsequent to the formation of primer extension products. Thus, the detectable complex is captured on the solid support material for convenient detection. A diagnostic test kit comprises the primers, an immobilized receptor, a polymerization agent and the deoxyribonucleotide triphosphates needed for carrying out the amplification and detection of the specific nucleic acid sequence.

# DIAGNOSTIC KIT AND METHOD USING A SOLID PHASE CAPTURE MEANS FOR DETECTING NUCLEIC ACIDS

The present invention relates to a diagnostic test kit and a method for detecting one or more specific nucleic acid sequences using oligonucleotide primers, a labeled probe and amplification procedures. The method includes a unique procedure for capturing the amplification products prior to detection. The present invention can be used in forensic investigations, and the detection of genetic disorders and infectious diseases.

Nucleic acid probe technology has developed rapidly in recent years as researchers have discovered its value for detection of various diseases, organisms or genetic features which are present in very small quantities in a test sample. The use of probes is based upon the concept of complementarity. For example, DNA is double-stranded, the strands bound to each other by hydrogen bonds between complementary nucleotides (also known as nucleotide pairs).

The DNA complex is normally stable, but the strands can be separated (or denatured) by conditions which disrupt the hydrogen bonding. The released single strands will reassociate only with another strand having a complementary sequence of nucleotides. This hybridization process can occur in solution or on a solid substrate. RNA is usually single-stranded and may not require denaturation. It will also hybridize with another strand or portion thereof which has a complementary sequence of nucleotides.

A target nucleic acid sequence of the DNA or RNA of a target organism or cell may be only a small portion of the total strand, so that it is very difficult to detect its presence using most known labeled probes. Much research has been carried out to overcome this problem including improvements in probe sensitivity and synthesis of nucleic acids.

A significant advance in the art is the process described in U.S. Patent 4,683,202. Without going into extensive detail regarding that process, it is an amplification technique wherein primers are hybridized to nucleic acid templates in the presence of a polymerization agent (such as a polymerase) and four nucleotide triphosphates, and extension products are formed from the primers. These products are denatured and used as templates in a cycling reaction which amplifies the number and amount of existing nucleic acids to facilitate their subsequent detection. The amplification process of U.S. Patent 4,683,202 can be carried out cyclically as many times as desired to produce a larger quantity of detectable material from a small amount of target nucleic acid sequence.

Once the target sequence has been adequately amplified to detectable quantities, the mode of detection is important. Many techniques for detection are described in the noted patent (Col. 15) including the use of probes labeled with radioisotopes, biotin or enzymes (linked to the probe through a biotin-avidin linkage, see Col. 26) or gel electrophoresis.

While there are advantages inherent in using the labeled primers known in the art, there is a need for further improvement, namely to simplify the construction of labeled primers and thereby facilitate the detection process.

The present invention represents an important improvement in the amplification and detection of nucleic acids using labeled primers.

Thus, the present invention provides a method for the detection of a specific nucleic acid sequence of a predetermined nucleic acid, the sequence having two complementary strands, the method comprising:

A. contacting a specimen suspected of containing the predetermined nucleic acid with first and second oligonucleotide primers complementary to the strands of a specific nucleic acid sequence, at least the first primer being labeled with a specific binding ligand,
so as to form a mixture of hybridized products of the first and second primers and the complementary strands,

B. forming first and second extension products of the primers in the hybridized products, which extension products, when separated from their complements, can serve as templates for synthesis of the extension products of the primers,

C. separating the primer extension products from the templates on which they were synthesized,

D. contacting the separated primer extension products and the specimen with additional first and second primers, resulting in amplification of the specific nucleic acid sequence to form complementary products,

E. separating the primer extension products from the complementary products formed in step D,

F. contacting the first primer extension product separated in step E with a detectably labeled oligonucleotide probe which is complementary thereto to form a product of the labeled probe and the first primer extension product, and

G. detecting the complementary product formed in step F as an indication of the presence of the predetermined nucleic acid in the specimen,

provided that prior to detection step G, the specific binding ligand of the first primer is complexed with a receptor therefor which is bound to a solid support material.

This invention also provides an aqueous suspension of polymeric particles to which are bound an oligonucleotide primer,

the suspension characterized wherein the primer is attached to the particles through a specific binding complex of a specific binding ligand and its receptor.

Moreover, a diagnostic test kit comprises:

a) first and second oligonucleotide primers complementary to the separate strands of a predetermined nucleic acid sequence,

b) a receptor for the specific binding ligand,

c) a polymerization agent for primer extension, and

d) the deoxyribonucleotide triphosphates dATP, dCTP, dGTP and dTTP, the kit characterized wherein at least one of the primers is labeled with a specific binding ligand, and the receptor is bound to a solid support material, the receptor provided in the kit either in separate packaging or as complexed with the specific binding ligand on one of the primers.

The present invention provides a means for rapidly detecting genetic materials which are generally present in a cell or organism in extremely small quantities. Moreover, the method of the present invention is adaptable to commercial automated detection procedures.

These advantages are achieved by using oligonucleotide primers which are labeled for "capture" or immobilization. The label on one of the primers is a specific binding ligand which specifically complexes with its receptor which is bound to a solid support of some type. Detection is then achieved using a detectably labeled probe complementary to the primer extension products formed in the method of the invention. The capture means described herein will provide an efficient and effective means for separating the amplified extension products from all other materials for detection. More than one specific nucleic acid sequence can be detected using the present invention, as described in more detail below.

In one embodiment, complexation of specific binding ligand and receptor is achieved before the primer extension products are formed. This embodiment utilizes an aqueous suspension of polymeric particles to which are bound an appropriate oligonucleotide primer. The primer is attached to the particles through a specific binding complex of a specific binding ligand and its receptor.

In other embodiments, the extension products are formed and amplification is carried out prior to capture. More details of these embodiments are provided below.

As used herein in referring to primers, probes or oligomer fragments to be detected, the term "oligonucleotide" refers to a molecule comprised of two or more deoxyribonucleotides or ribonucleotides, and preferably more than three. The exact size is not critical but depends upon many factors including the ultimate use or function of the oligonucleotide. The oligonucleotide may be derived synthetically or by cloning.

The term "primer" refers to an oligonucleotide, whether naturally occurring or synthetically produced, which is capable of acting as a point of initiation of synthesis when placed under conditions in which synthesis of a primer extension product complementary to a nucleic acid strand is induced. Such conditions include the presence of nucleotides (such as the four standard deoxyribonucleotide triphosphates) and an agent for polymerization such as a DNA polymerase, and suitable temperature and pH.

In one embodiment, the primer contains a double-stranded, labeled nucleic acid region adjacent to a single-stranded region. The single-stranded region contains a nucleic acid sequence which is sufficiently complementary to the template strand to hybridize therewith. The double-stranded region, or tail, of the primer can be labeled with a detectable moiety which is capable of producing a detectable signal or which is useful in capturing or immobilizing the extension product. Further details regarding such primers, useful labels and methods of preparation are known in the art.

In other and preferred embodiments, the primer is entirely single-stranded. Preferably, the primer is a single-stranded oligodeoxyribonucleotide. It must be sufficiently long to prime the synthesis of extension products in the presence of the polymerization agent, but its exact size will vary depending upon the use contemplated, the complexity of the target sequence, reaction temperature and the source of the primer. Generally, each primer used in this invention will have from 15 to 40 nucleotides, and preferably, it has from 20 to 25 nucleotides.

The primers used in the present invention are selected to be "substantially" complementary to the different strands of each specific sequence to be amplified. This means that they must be sufficiently complementary to hybridize with their respective strands to form the desired hybridized products. Noncom-

3

plementary bases may be incorporated therein as long as they do not interfere with hybridization and formation of extension products. Preferably, the primers have exact complementarity to obtain the best results in amplification efficiency.

The present invention is directed to the detection of one or more specific nucleic acid sequences present in the same or different predetermined nucleic acids in a test specimen. Such samples can include cellular or viral material, hair, body fluids or other materials containing genetic DNA or RNA which can be detected. While the primary purpose of such detection would be diagnostic in nature, the invention could also be used to improve the efficiency of cloning DNA or messenger RNA, or for obtaining large amounts of the desired sequence from a mixture of nucleic acids resulting from chemical synthesis.

The present invention involves a chain reaction for producing, in exponential quantities relative to the number of reaction steps involved, at least one specific nucleic acid sequence. The product will be a discrete nucleic acid duplex with termini corresponding to the ends of the specific primers employed. Any source of nucleic acid, purified or not, can be utilized as the starting material provided it contains or is suspected of containing the specific nucleic acid sequence targeted for detection. A mixture of nucleic acids can be employed if desired. The sequence to be duplicated can be a fragment or the entire nucleic acid. Moreover, more than one nucleic acid sequence can be amplified simultaneously by using a specific set of primers and labeled probes for each sequence to be amplified. The sequences can be in the same or different nucleic acids. Each set of primers has at least one primer which has a specific binding ligand particular to that set, so that each sequence can be detected individually or collectively. Both primers of each set can be labeled with a specific binding ligand if desired.

Nucleic acids can be obtained from various sources including plasmids, naturally occurring DNA or RNA from any source (such as bacteria, yeast, viruses, plants and higher animals, humans). It may be extracted from various tissues including blood, tissue material or other sources known in the art using known procedures. The present invention is particularly useful for the detection of nucleic acid sequences found in viruses or cells of any organism, such as in bacterial DNA, viral RNA, or DNA or RNA found in bacterial or viral infected cells. This invention is particularly useful for the detection of DNA from cells infected by HIV-I or other retroviruses.

Primers useful herein can be obtained from a number of sources or prepared using known techniques and equipment, including for example, an ABI DNA Synthesizer or a SAM-I Synthesizer and known methods for their use. Naturally occurring primers isolated from biological sources are also useful (such as restriction endonuclease digests).

At least one of the primers (or sets thereof) used in the practice of the present invention is labeled with a specific binding ligand. The term "labeled" refers to the fact that the ligand is attached to this primer in a manner such that it will not readily be detached. In most instances, this means that the ligand is chemically attached to the primer directly or indirectly. Methods for doing this will vary depending upon the moiety so attached. The specific binding ligand can be biotin or a derivative thereof, avidin, streptavidin or a derivative thereof, a lectin, a sugar, a protein, a hapten, a drug, or an immunological species, such as an antibody or an antigenic material. By immunological species is meant either: (1) any substance which, when presented to an immunocompetent host, will result in the production of a specific antibody capable of binding with that substance, or (2) the antibody so produced, which species participates in an antigen-antibody reaction in the use thereof.

All of these ligands have reactive groups which can be used to attach it to the primer either directly or through intermediate linking groups. For example, biotin can be attached to oligonucleotides using the standard procedures described by Kempe et al, Nucleic Acid Res., 13, pp. 45-57 (1985). Another attachment technique is by use of an alkylating intercalation moiety as described in U.S. Patent 4,582,789.

A teaching of how to derivatize DNA for attachment of biotin or other ligands is provided in WO-A-0 002 931 (1989). Briefly, this means of biotin attachment is achieved by condensing a N-hydroxysuccinimide activated ester of biotin with the derivatized oligonucleotide described in the noted publication.

A lectin can be attached to a primer by using standard protein coupling methods and derivatized oligonucleotides.

An immunological species (that is, an antibody, antigenic material, hapten and others) can be attached by methods similar to those noted above for lectins and biotin. Such attachment techniques are well known in the art.

The specific binding ligand is so defined because it will complex specifically and preferentially with a corresponding receptor molecule to form a specific binding complex. In most instances, this complexation is irreversible, although under certain conditions, complex formation may be reversible. This complex may or may not be soluble at the time of complexation, but eventually in the practice of this invention, it will become insoluble at some point (if not at the time of complexation) in order to separate the complexed product of amplification from uncomplexed materials.

Receptor molecules are those which correspondingly complex only with the specific binding ligands, and would be readily apparent once the ligand of interest is chosen. For example, if the ligand is biotin or a derivative thereof, the receptor molecule is avidin, streptavidin or a derivative thereof. If the moiety is an antigenic material, the receptor is an antibody. If the ligand is an antibody, the receptor can be an antigenic material to which the antibody is directed, or an anti-antibody. The receptor for a lectin is a sugar. Other ligand-receptor combinations could be readily determined by one of ordinary skill in the art having the teaching herein available. Because there are a number of specific binding ligand-receptor combinations, the present invention can also be used to detect a multiplicity (two or more) of specific nucleic acid sequences by using different sets of primers, each set having a primer with a different specific binding ligand which will not interfere with the corresponding receptor-ligand binding of the other ligands. Thus, a biotin-avidin complex can be used for detection of one specific nucleic acid sequence while a lectin-sugar complex could be used simultaneously to detect another specific sequence.

The present invention is useful for detection of a specific nucleic acid having two complementary strands. Most nucleic acid sequences of interest already are double-stranded, such as those found in DNA. However, single-stranded nucleic acid sequences, such as mRNA, can be similarly detected after it is converted to a double-stranded sequence using reverse transcriptase.

A specific nucleic acid sequence is produced using the nucleic acid containing that sequence as a template. If the acid contains two strands, it is necessary to separate the strands (called dehybridization or denaturation), either as a separate step or simultaneously with the formation of primer extension products. Denaturing can be accomplished using any suitable physical, chemical or enzymatic means as described in the art. Heating to a suitable temperature is a preferred means.

Once the separated strands are available for use, synthesis of additional nucleic acid strands can be carried out using two or more primers (at least one of which is labeled as described above) in a buffered aqueous solution at a pH of from 7 to 9. Preferably, a molar excess of the two primers is added to the buffered solution, and specific amounts are taught in the art. The deoxyribonucleotide triphosphates dATP, dCTP, dGTP and dTTP are also added to the synthesis mixture in adequate amounts and the resulting solution is heated to 90-100°C for up to 10 minutes, and preferably from 1 to 4 minutes. After this heating, the solution is preferably cooled to room temperature, and an appropriate agent for inducing (or catalyzing) the formation of primer extension products is introduced. This inducing agent is generally known in the art as a polymerization agent. Reaction to form these products is carried out under known conditions (generally from room temperature to that temperature at which polymerization no longer occurs).

The polymerization agent may be any compound, or combination of reagents, which will function to accomplish the synthesis of primer extension products, including enzymes (for example, E. coli DNA polymerase I, T4 DNA polymerase, Klenow polymerase, reverse transcriptase and others known in the art). Particularly useful enzymes are thermally stable enzymes, cloned or naturally occurring, such as those obtained from various Thermus bacterial species. Other polymerization agents are described in U.S. Patent 4,683,202.

Preferred thermal-stable enzymes are DNA polymerases from Thermus aquaticus as described in EP-A-0 258 017. Those polymerases generally have a molecular weight of 86,000-90,000 daltons. Other useful enzymes are described by Rossi et al, Syst. Appl. Microbiol. 7(2-3), pp. 337-341, 1986. Many useful polymerases are commercially available. Generally, the synthesis of extension products will be initiated at the 3′ end of each primer and proceed in the 5′-3′ direction along the template until synthesis is terminated. Some polymerization agents (for example, reverse transcriptase) may proceed in the 3′ to 5′ direction along the template.

The newly formed primer extension products comprising the newly synthesized strands and their respective primers form double-stranded molecules with the initial target strands which are used in the succeeding steps of the method. These strands are then separated by denaturation as described above to provide single-stranded molecules, onto which new nucleic acids are synthesized as described above. Additional reagents may be needed to keep the amplification procedure going, after which most of the extension products will consist of the specific nucleic acid sequence bounded by the two primers (that is, complementary products).

The steps of strand separation and extension product synthesis can be repeated as often as needed to produce the desired quantity of the specific nucleic acid needed for the use, for example detection. Generally, the sequence of steps is repeated at least once, and preferably at least 10 to 30 times.

When it is desired to produce more than one specific nucleic acid from the first nucleic acid or a mixture thereof, the appropriate number of sets of primers are used in the general procedure described above.

At any point in the method of this invention after the generation of at least one primer extension product, that product can be hybridized with a detectably labeled probe (described below). This contact of probe and extension product can occur before or after complexation of ligand and receptor.

Generally, once a desired amount of the nucleic acid sequence of interest has been generated and the primer extension products are separated for a last time, the first primer extension product (that is, the one formed from labeled primer) is contacted with an oligonucleotide probe which is labeled for detection and is complementary thereto to form a product. The probe is a nucleic acid sequence which is complementary with the target nucleic acid sequence. The probes can be of any suitable length of nucleic acids, but preferably it is from 10 to 30 nucleic acids. It is labeled (commonly at the 5' end) with any suitable detectable material which will not interfere with the complexation of the specific binding ligand and its receptor. Procedures for attaching labels and preparing probes is well known in the art, for example, as described by Agrawal et al, Nucleic Acid Res., 14, pp. 6227-45 (1986), and in the references noted above for attaching a specific binding ligand to a primer. Useful labels include radioisotopes, electron-dense reagents, chromogens, fluorogens, phosphorescent moieties, ferritin and other magnetic particles, chemiluminescent moieties and enzymes (which are preferred). Useful enzymes include, glucose oxidase, peroxidase, uricase, alkaline phosphatase and others known in the art. Substrates and dye forming compositions for such enzymes are well known.

In a particularly preferred embodiment, the label is peroxidase, and at some point in the assay, hydrogen peroxide and suitable dye-forming compositions are added to provide a detectable dye. For example, useful dye-providing reagents include tetramethylbenzidine and derivatives thereof, and leuco dyes, such as triarylimidazole leuco dyes (as described in U.S. Patents 4,089,747, or other compounds which react to provide a dye in the presence of peroxidase and hydrogen peroxide. Particularly useful dye-providing compositions are described in EP-A-0 308 236.

Detection of the presence of the probe which is in the complementary product can be achieved using suitable and known detection equipment and procedures. Certain probes may be visible to the eye without the use of detection equipment. It is also useful for the method to be carried out in a suitable container. The most crude container would be a test tube, flask or beaker, but more sophisticated containers have been fashioned in order to facilitate automated procedures for performing the method. For example, a cuvette constructed to provide certain temperature characteristics during the practice of the method is described and claimed in copending EP-A-0 318 255. Other useful containers could be suitably fashioned for automated or single use of the method of this invention.

In order for the probe in the complementary product to be detected, it is often important for the complementary product to be separated from the other materials in the reaction medium. This is done by allowing the specific binding ligand of the first primer (now part of the first extension product or the complementary product) to complex with a receptor therefor, which receptor is bound to a solid support material. The resulting insolubilized complexed product can be separated from uncomplexed materials by filtration, centrifugation or other suitable separation techniques.

One particularly useful separation means are microporous filter membranes such as the polyamide membranes (for example as Loprodyne™ or Biodyne™ membranes). They can be used uncoated or precoated with surfactants or other materials which facilitate the analytical procedures. In one embodiment, the membrane itself can have the receptor molecules attached thereto (by absorption or covalent bonds) for capturing the first primer extension products.

The membranes can be used as a separate substrate with suitable containers for carrying out other steps of the assay. Preferably, however, it is mounted as part of a test device. Various test devices are known in the art including those described in U.S. Patents 3,825,410, 3,888,629, 3,970,429 and 4,446,232. Particularly useful devices are described in EP-A-0 308 231.

The complexed product then is detectable by means noted above which depend upon the type of probe used. The complexation must occur prior to the detection of the complementary product, but the timing of complexation prior to that time is not critical. As will be discussed in more detail below, complexation can occur either before or after formation of the primer extension products.

The receptor is bound to a solid support material of some type which facilitates separation of complexed product and uncomplexed materials. For example, the solid support can be a microtiter plate, test tube, beaker, beads, film, membrane filters, filter papers, gels, magnetic particles or glass wool. It can be made of a number of materials including glass, ceramics, metals, naturally occurring or synthetic polymers, cellulosic materials, filter materials and others readily apparent to one of ordinary skill in the art. Particularly useful solid support materials are polymeric beads generally having an average particle size of from 0.1 to 10 μmeters. The receptor can be bound to the support material in any suitable manner including absorption and covalent attachment. Covalent attachment is preferred in most instances. These attachment means are well known in the art.

In a preferred embodiment, the solid support material is prepared from polymeric materials which have pendant reactive groups which can be reacted directly or indirectly with reactive receptor molecules. By "direct" reaction is meant that the pendant reactive groups react directly with reactive amino or sulfhydryl groups of the receptor molecules without linking or intermediate molecules. Alternatively, either or both the receptor or specific binding ligand can be chemically modified to provide reactive sites for attachment as long as such modification does not adversely affect the sites where the ligand and receptor complex with each other. For example, biotin may not be attachable directly to the solid support, but suitable biotin derivatives having suitable reactive groups (such as succinimidooxycarbonyl, maleimidooxycarbonyl or N´- bromoacetylhydrazinocarbonyl) can be attached to a support pretreated with a protein such as casein to provide amine groups that react with the biotin derivative. In addition, the receptor molecules can be attached to the solid support "indirectly" through a linking moiety which can be a protein, peptide, polypeptide, diamine or dimercaptan.

In preferred embodiments, at least the first primer is labeled with biotin or a biotin derivative, and the receptor attached to the solid support is avidin or an avidin derivative. Avidin derivatives include streptavidin, succinylated avidin and monomeric avidin. Biotin derivatives include biotin-$\epsilon$-N-lysine, biocytin hydrazide, amine or sulfhydryl derivatives of 2-iminobiotin and biotinyl-$\epsilon$-aminocaproic acid hydrazine. Other biotin derivatives, such as biotin-N-hydroxysuccinimide ester, biotinyl-$\epsilon$-aminocaproic acid-N-hydroxysuc-cinimide ester, sulfosuccinimidyl-6-(biotin amido)hexanoate, N-hydroxysuccinimideiminobiotin, biotinbromoacetylhydrazide, p-diazobenzoyl biocytin and-3-(N-maleimidopropionyl)biocytin, can also be attached to linking proteins after such proteins have been suitably attached to the solid support.

The solid support can have suitable reactive groups for attaching the receptor molecules. Such groups can be a part of the support material or result from a chemical treatment of the material. Useful reactive groups include carboxy, amino, sulfhydryl, aldehyde, activated 2-substituted ethylsulfonyl, vinylsulfonyl, active halogen atoms, nitroaryl and others readily apparent to one skilled in the art.

Particularly useful solid support materials are polymeric particles derived from one or more $\alpha,\beta$-ethylenically unsaturated polymerizable monomers having one or more of the following reactive groups: activated 2-substituted ethylsulfonyl, vinylsulfonyl or active halogens. Such particles generally have an average particle size greater than 0.01 $\mu$meters. Preferably, the average particle size is in the range of from 0.1 to 10 $\mu$meters.

Further details about such preferred polymeric particles, including useful monomers, methods of preparing them and attachment of receptor molecules, are provided in copending EP-A-0 302 715.

In one embodiment of this invention, a method for the detection of a specific nucleic acid sequence of a predetermined nucleic acid, the sequence having two complementary strands, comprises:

A. contacting a specimen suspected of containing the predetermined nucleic acid with

(1) first and second oligonucleotide primers complementary to the strands of the specific nucleic acid sequence, at least the first primer being labeled with a specific binding ligand, and

(2) a solid support material having a receptor for the specific binding ligand bound thereto,

so as to form a mixture of hybridized products of the first and second primers and the complementary strands, at least the first primer bound to the support material through a complex of the specific binding ligand and the receptor,

B. forming first and second extension products of the primers in the hybridized products, which extension products, when separated from their complements, can serve as templates for synthesis of the extension products of the primers,

C. separating the primer extension products from the templates on which they were synthesized, the first primer extension product being insoluble,

D. contacting the separated primer extension products and the specimen with additional first and second primers, resulting in amplification of the specific nucleic acid sequence to form complementary products,

E. separating the primer extension products from the complementary products formed in step D,

F. contacting the insoluble first primer extension product separated in step E with a detectably labeled oligonucleotide probe which is complementary thereto to form a product of the labeled probe and insoluble first primer extension product, and

G. detecting the insoluble complementary product formed in step F as an indication of the presence of the predetermined nucleic acid in the specimen.

This embodiment may be used to detect a specific nucleic acid sequence in the gag region of HIV-I DNA, in HLA DNA or in human $\beta$-globin DNA. In step A, a specimen suspected of containing HIV-I DNA, HLA DNA or human $\beta$-globin DNA is contacted with (1) first and second oligonucleotide primers complementary to the strands of a specific nucleic acid sequence in the gag region of HIV-I DNA, in HLA DNA or in human β-globin DNA, respectively, at least the first primer being labeled with biotin, and (2) polymeric particles having an average particle size of from 0.1 to 10 μmeter and having avidin covalently bound thereto. Preferably, the oligonucleotide probe used in step F is enzyme-labeled and the insoluble complementary product formed in step F is detected by contacting it with a composition which provides a dye in the presence of the enzyme.

In this embodiment, the first primer is insolubilized prior to formation of the primer extension products. Where the solid support material is a polymeric particle, the first primer can be supplied in an aqueous suspension of particles to which the primer is bound through the specific binding complex formed from the specific binding ligand of the primer and the receptor attached to the particles. This suspension can contain other useful addenda including buffers, suspending agents, stabilizers or emulsifiers. The percent solids is generally at least 0.01, and preferably from 0.1 to 25, weight percent of the suspension. Alternatively, the first primer having the specific binding ligand and the insoluble receptor can be supplied separately and mixed prior to use in the method to complex primer to the insoluble receptor.

In another embodiment of this invention, a method for the detection of a specific nucleic acid sequence of a predetermined nucleic acid, the sequence having two complementary strands, comprises:

A. contacting a specimen suspected of containing the predetermined nucleic acid with first and second oligonucleotide primers complementary to the specific nucleic acid sequence strands, at least the first primer being labeled with a specific binding ligand,

so as to form a mixture of hybridized products of the first and second primers and the complementary strands,

B. forming first and second extension products of the primers in the specific hybridized products, which extension products, when separated from their complements, can serve as templates for synthesis of the extension products of the primers,

C. separating the primer extension products from the templates on which they were synthesized,

D. contacting the separated primer extension products and the specimen with additional first and second primer, resulting in amplification of the specific nucleic acid sequence to form complementary products,

E. separating the primer extension products from the complementary products formed in step D,

F. contacting the first primer extension product separated in step E with a detectably labeled oligonucleotide probe which is complementary thereto to form a product of the labeled probe and first primer extension product,

G. contacting the complementary product formed in step F with a solid support material to which is bound a receptor for the specific binding ligand of the first primer, thereby insolubilizing the probe-first primer complementary product through complexation of the specific binding ligand and receptor therefor, and

H. detecting the insolubilized complementary product formed in step G as an indication of the presence of the predetermined nucleic acid in the specimen.

This embodiment may be used to detect a specific nucleic acid sequence in the gag region of HIV-I DNA, in HLA DNA or in human β-globin DNA. In step A, a specimen suspected of containing HIV-I DNA, HLA DNA or human β-globin DNA is contacted with first and second oligonucleotide primers complementary to the strands of a specific nucleic acid sequence in the gag region of HIV-I DNA, in HLA DNA or in human β-globin DNA, respectively, at least the first primer being labeled with biotin. Preferably, the oligonucleotide probe used in step F is enzyme-labeled and the solid support material in step G comprises polymeric particles having an average particle size of from 0.1 to 10 μmeter and having avidin covalently bound thereto. The insolubilized complementary product formed in step G is detected by contacting it with a composition which provides a dye in the presence of the enzyme.

The method described herein can be used to provide the detection or characterization of specific nucleic acid sequences associated with infectious diseases, genetic disorders or cellular disorders such as cancers. It may also be used in forensic investigations and DNA typing. For purposes of this invention, genetic diseases include specific deletions or mutations in genomic DNA from any organism, such as sickle cell anemia, cystic fibrosis, α-thalassemia, β-thalessemia and others readily apparent to one skilled in the art. Various infectious diseases can be diagnosed by the presence in a clinical sample of small quantities of specific DNA sequences characteristic of the organism, whether it be a yeast, bacterium or virus. Such bacteria which can be detected include, but are not limited to, Salmonella, Chlamydia, Gonorrhea, Shigella and Listeria. Viruses which are detectable include, but are not limited to, herpes, hepatitis and retroviruses such as HTLV-I and HIV-I. Protozoan parasites, yeasts and molds are also detectable. Other detectable species would be readily apparent to one skilled in the art. The invention is particularly useful for the

8

detection of the presence of retroviruses, such as HIV-I, in test samples.

The diagnostic test kit of this invention has been generally described above. Critical components in the kit include first and second oligonucleotide primers, at least one of which is labeled with a specific binding ligand, the primers being complementary to the strands of a predetermined nucleic acid sequence of interest to the user. The kit may contain a set of primers for each nucleic acid sequence of interest.

The kit also contains a receptor for the specific binding ligand, which receptor is covalently bound to a solid support material (as described above). The receptor can be provided in the kit either in separate packaging, or complexed with the specific binding ligand of one the primers.

Moreover, the kit comprises an agent for inducing primer extension, as described above, such as a polymerase, and each of the deoxyribonucleotide triphosphates dATP, dCTP, dGTP and dTTP, generally in separate containers.

The kit components are packaged in a suitable manner, and can be included with a number of optional components such as enzyme substrates, dye-providing compositions, pipettes, cuvettes, instructions, buffers, wash solutions, diluents and other reagents, materials and equipment which may be useful in practicing the present invention. These additional components are well known in the art.

The following examples are included to illustrate the practice of this invention, and are not meant to be limiting in any way.

In these examples, the primers and probes used therein were prepared as follows and had the indicated nucleic acid sequences. As is standard in the art, the sequences are identified by letters identifying the individual nucleotide bases, that is adenine(A), thymine(T), guanine(G) and cytosine(C).

## Primers (+ and -) for Examples 1-3:

The primers for the HIV-I DNA target sequence noted below have the following nucleotide sequences, X-TTTGGTCCTTGTCTTATGTCCAGAATGC and X-ATAATCCACCTATCCCAGTAGGAGAAAT, respectively, wherein X represents a biotintetraethylene glycol spacer arm prepared and attached as described in WO-A-0 002 931 (1989).

## HIV-I target for Examples 1-3:

The DNA target detected in Examples 1-3 was the 180 nucleotide segment of the gag region (core protein) of the HIV-I genome cloned into a derivative of M13 vector, and prepared using standard procedures.

## Probe for Examples 1-3:

The DNA probe used in Examples 1-3 had the following nucleotide sequence Y-ATCCTGGGATTAAATAAAATAGTAAGAATGT wherein Y represents horseradish peroxidase which was linked to the oligonucleotide using a mal sac HNSA propyl thiol linker arm according to the teaching of WO-A-0 002 932 (1989).

## Primers (+ and -) for Example 4:

The primers for the Human Leukocyte Antigen (HLA) DNA target sequence noted below have the following nucleic acid sequences, X-GTGCTGCAGGTGTAAACTTGTACCAG and X-CACGGATCCGGTAGCAGCGGTAGAGTTG, respectively, wherein X is an defined above.

## HLA target for Example 4:

The DNA target detected in Example 4 was DNA extracted from a human whole blood sample.

## Probe for Example 4:

EP 0 370 694 A2

The DNA probe used in Example 4 had the following nucleotide sequence Y-TTCCGCAGATTTAGAAGAT, wherein Y represents horseradish peroxidase linked to the oligonucleotide using a mal sac HNSA tetraethylene glycol thiol linker arm according to the teaching of WO-A-0 002 932 (1989).

Primers (+ and -) for Example 5:

The primers for the human β-globin DNA target sequence noted below have the following nucleic acid sequences, X-ACACAACTGTGTTCACTAGC and X-CAACTTCATCCACGTTGACC, respectively, wherein X is as defined above.

Human β-globin target for Example 5:

The DNA target detected in Example 5 was β-globin DNA extracted from a human whole blood sample.

Probe for Example 5:

The DNA probe used in Example 5 had the following nucleotide sequence Y-CCTGAGGAGGAAGTCT wherein Y is horseradish peroxidase linked to the oligonucleotide using a mal sac HNSA tetraethylene glycol thiol linker arm according to the teaching of WO-A-0 002 932 (1989).

The leuco dye composition used in the following examples had the following components:
2-(4-hydroxy-3-methoxyphenyl)-4,5-bis(4-methoxyphenyl)imidazole leuco dye (0.008 weight %)
4' hydroxyacetanilide (5 mmolar),
poly(vinylpyrrolidone) (1 weight %),
diethylenetriaminepentaacetic acid (10 μmolar),
hydrogen peroxide (10 mmolar), and
monobasic sodium phosphate (10 mmolar).

Example 1: Detection of DNA from HIV-I

This example demonstrates the practice of the present invention to detect the presence in a sample of a specific nucleic acid sequence which is unique to HIV-I DNA.

The following reaction mixture (100 μl) was prepared:
Primers (+ and -)(1 μmolar),
Target nucleic acid sequence from HIV-I DNA (M13 clone) ($10^{-13}$ molar),
Potassium chloride (0.05 molar),
Tris(hydroxymethyl)aminomethane hydrochloride buffer (pH 8, 0.01 molar),
Deoxynucleotide triphosphates (dNTP, 1.5 mmolar each, 6 mmolar total),
DNA polymerase isolated from a commercially available Thermus aquaticus (2.5 units where 1 unit corresponds to 10 nmoles dNTP incorporated in the primer extension product in 30 minutes at 74° C),
Magnesium chloride (10 mmolar),
Gelatin (100 μg/ml),
Avidin covalently bound to poly[styrene-co-m,p-(2-chloroethylsulfonylmethyl)styren e](90:10 molar monomer ratio) beads (2.6 μmeters average particle size) (0.12% or 0.24% solids).

Two control mixtures were also formed:
Control A having the same reagents as above but omitting the avidin-bead reagent, and Control B having the same reagents but omitting the DNA polymerase.

Each mixture was placed into a polypropylene microcentrifuge tube, primer extension products were formed, and amplification promoted using 30 consecutive thermal cycles as follows:

10

| 70°C rising to 94°C | 1 minute |
| 94°C | 0.5 minute (denature) |
| 94°C lowering to 50°C | 1.25 minutes |
| 50°C | 0.5 minute (hybridize) |
| 50°C rising to 70°C | 0.75 minute |
| 70°C | 1 minute (extend) |

After amplification through the 30 thermal cycles, 5 μl aliquots of each mixture were removed from the tubes, heat denatured (5 minutes at 95°C), and subjected to hybridizing conditions (30 minutes at 42°C) in the presence of an oligonucleotide probe (described above) conjugated to horseradish peroxidase (1 pmole).

Microporous membranes situated in disposable test devices like those described in EP-A-0 308 231 (noted above) were pretreated by incubating them for 10 minutes at room temperature with Denhardt's solution [1 g Ficoll from Sigma Chemical Co., 1 g poly(vinylpyrrolidone) and 1 g bovine serum albumin in 100 ml water], and SSPE solution (43.5 g sodium chloride, 6.9 g sodium phosphate, 1.85 g ethylenediaminetetraacetic acid in 1 liter water, adjusted to pH 7.4), followed by draining. Both Denhardt's Solution and SSPE solution are known in the art (Maniatis, Fritsch and Sambrook, Molecular Cloning, A Laboratory Manual, Cold Harbor Spring Laboratory, 1982).

Samples of the test and control mixtures described above were added to the disposables followed by washing five times with a wash solution consisting of 50 μl of SSPE solution (1.75 g sodium chloride, 0.276 g sodium phosphate and 0.074 g ethylenetriaminetetraacetic acid in 1 liter of water, adjusted to pH 7.4) and 0.02% (by weight) of Triton™ X-100 nonionic surfactant heated to 55°C. The leuco dye composition described above was added (100 μl), and the color on each membrane was allowed to develop for 5 minutes followed by visual reading and grading from 0 to +5, zero representing no color, and +5 being highly colored.

The results are shown in the following Table and demonstrate that the method of the present invention was useful in detecting the HIV-I DNA nucleic acid sequence of interest. All of the results were compared to the color detected from the Controls. The results were substantiated with density readings from a spectrophotometer.

TABLE

| Reagent Mixture | Color Reading |
| --- | --- |
| Control A | 0 |
| Control B | 0 |
| Invention (0.12% beads-avidin) | +1.5 |
| Invention (0.24% beads-avidin) | +2 to +3 |

Examples 2 & 3: Detection of HIV-I DNA with Insoluble Reagent Provided After Amplification

These examples followed the procedure of Example 1 except that insolubilization occurred after the amplification cycles had been carried out. In Example 2, the avidin was supplied as attached to polymeric particles as in Example 1, but in Example 3, avidin was supplied as attached to the nylon microporous membranes in the disposable test devices. Control mixtures were prepared and tested as in Example 1.

The Controls provided no visible color difference over background, but the methods of this invention provided a color reading of +4 for Example 2 and +5 for Example 3.

Example 4: Detection of HLA DNA Nucleic Acid Sequence

This example demonstrates the practice of the present invention to detect the presence in a sample of a specific nucleic acid sequence which is unique to Human Leukocyte Antigen (HLA) DNA.

The following reaction mixture (100 μl) was prepared:

Primers ( + and -)(0.2 μmolar),

Target nucleic acid sequence from whole blood (20 μl extract/100 μl reaction mixture),

Potassium chloride (0.05 molar),

Tris(hydroxymethyl)aminomethane hydrochloride buffer (pH 8, 0.01 molar),

Deoxynucleotide triphosphates (dNTP, 0.175 mmolar each, 0.7 mmolar total),

DNA polymerase isolated from Thermus aquaticus (2.0 units where 1 unit corresponds to 10 nmoles dNTP incorporated in the primer extension product in 30 minutes at 74°C),

Magnesium chloride (2.5 mmolar),

Gelatin (100 μg/ml),

Avidin covalently bound to poly[styrene-co-m,p-(2 chloroethylsulfonylmethyl)styren e](95.5:4.5 molar monomer ratio) beads (2.6 μmeters average particle size) (0.24% solids).

Two control mixtures were also formed:

Control A having the same reagents as above but omitting the avidin-bead reagent, and Control B having the same reagents but omitting the target nucleic acid sequence.

Each mixture was placed into a polypropylene microcentrifuge tube, primer extension products were formed, and amplification promoted using 30 consecutive thermal cycles as follows:

| 70°C rising to 95°C | |
| 95°C | 0.5 minute (denature) |
| 95°C lowering to 55°C | |
| 55°C | 0.5 minute (hybridize) |
| 55°C rising to 70°C | |
| 70°C | 1 minute (extend) |

After amplification through the 30 thermal cycles, 5 μl aliquots of each mixture were removed from the tubes, heat denatured (5 minutes at 95°C), and subjected to hybridizing conditions (5 minutes at 42°C) in the presence of an oligonucleotide probe (described above) conjugated to horseradish peroxidase (1 pmole).

Samples of the test snd control mixtures described above were added to the microporous membranes situated in disposable test devices, followed by washing five times with a wash solution consisting of 50 μl of SSPE solution (8.7 g sodium chloride, 1.4 g sodium phosphate, 0.37 g ethylenediaminetetraacetic acid in 1 liter water, adjusted to pH 7.4) and 0.5% (by weight) of sodium dodecyl sulfate heated to 55°C. The leuco dye composition described above was added (100 μl), and the color on each membrane was allowed to develop for 2 minutes followed by visual reading and grading from 0 to +5, zero representing no color, and +5 being highly colored.

The results are shown in the following Table and demonstrate that the method of the present invention was useful in detecting the HLA DNA nucleic acid sequence of interest. All of the results were compared to the color detected from the Controls.

TABLE

| Reagent Mixture | Color Reading |
| --- | --- |
| Control A | 0 |
| Control B | 0 |
| Invention (0.24% beads-avidin) | +1.5 |

Example 5: Detection of HLA DNA with an Insoluble Reagent Provided After Amplification

This example followed the procedure of Example 4 except that insolubilization occurred after the amplification cycles had been carried out and prior to addition of the primer extension products to the disposable test devices. The avidin was supplied as attached to polymeric particles as in Example 4. Control mixtures were prepared and tested as in Example 4.

The Controls provided no visible color difference over background, but the method of this invention provided a color reading of +2.5.

Example 6: Detection of Human $\beta$-globin DNA Nucleic Acid Sequence

This example demonstrates the practice of the present invention to detect the presence in a sample of a specific nucleic acid sequence which is unique to human $\beta$-globin DNA.

The following reaction mixture (100 $\mu$l) was prepared:

Primers (+ and -)(0.2 $\mu$molar),
Target nucleic acid sequence from whole blood (20 $\mu$l extract/100 $\mu$l reaction mixture),
Potassium chloride (0.05 molar),
Tris(hydroxymethyl)aminomethane hydrochloride buffer (pH 8, 0.01 molar),
Deoxynucleotide triphosphates (dNTP, 0.175 mmolar each, 0.7 mmolar total),
DNA polymerase isolated from Thermus aquaticus (2 units where 1 unit corresponds to 10 nmoles dNTP incorporated in the primer extension product in 30 minutes at 74° C),
Magnesium chloride (2.5 mmolar),
Gelatin (100 ug/ml),
Avidin covalently bound to poly[styrene-co-m,p-(2-chloroethylsulfonylmethyl)styrene](95.5:4.5 molar monomer ratio) beads (2.6 $\mu$meters average particle size) (0.24% solids).

Two control mixtures were also formed:
Control A having the same reagents as above but omitting the avidin bead reagent, and Control B having the same reagents but omitting the target nucleic acid sequence.

Each mixture was placed into a polypropylene microcentrifuge tube, primer extension products were formed, and amplification promoted using 30 consecutive thermal cycles as follows:

| 70° C rising to 95° C 95° C 95° C lowering to 55° C | 0.5 minute (denature) |
|---|---|
| 55° C 55° C rising to 70° C | 0.5 minute (hybridize) |
| 70° C | 1 minute (extend) |

After amplification through the 30 thermal cycles, 5 $\mu$l aliquots of each mixture were removed from the tubes, heat denatured (5 minutes at 95° C), and subjected to hybridizing conditions (5 minutes at 42° C) in the presence of an oligonucleotide probe (described above) conjugated to horseradish peroxidase (1 pmole).

Samples of the test and control mixtures described above were added to the microporous membranes situated in disposable test devices, followed by washing five times with a wash solution consisting of 50 $\mu$l of SSPE solution (8.7 g sodium chloride, 1.38 sodium phosphate, 0.37 g ethylenetriaminetetraacetic acid in 1 liter water, adjusted to pH 7.4) and 0.5% (by weight) of sodium dodecyl sulfate at room temperature. The leuco dye composition described above was added (100 $\mu$l), and the color on each membrane wss allowed to develop for 2 minutes followed by visual reading and grading from 0 to +5, zero representing no color, and +5 being highly colored.

The results are shown in the following Table and demonstrate that the method of the present invention was useful in detecting the human $\beta$-globin DNA nucleic acid sequence of interest. All of the results were compared to the color detected from the Controls.

13

TABLE

| Reagent Mixture | Color Reading |
|---|---|
| Control A | 0 |
| Control B | +0.5 |
| Invention (0.24% beads-avidin) | +2.5 to +3 |

Examples 7: Detection of Human β-globin DNA with Insoluble Reagent Provided After Amplification

These examples followed the procedure of Example 6 except that insolubilization occurred after the amplification cycles had been carried out and prior to addition of the primer extension products to the disposable test devices. The avidin was supplied as attached to polymeric particles as in Example 6. Control mixtures were prepared and tested as in Example 6.

The Controls provided no visible color difference over background, but the methods of this invention provided a color reading of +3.5 to +4.

Claims

1. A method for the detection of a specific nucleic acid sequence of a predetermined nucleic acid, the sequence having two complementary strands, the method comprising:

A. contacting a specimen suspected of containing a predetermined nucleic acid with first and second oligonucleotide primers complementary to the strands of the specific nucleic acid sequence, at least the first primer being labeled with a specific binding ligand,

so as to form a mixture of hybridized products of the first and second primers and the complementary acid strands,

B. forming first and second extension products of the primers in the hybridized products, which extension products, when separated from their complements, can serve as templates for synthesis of the extension products of the primers,

C. separating the primer extension products from the templates on which they were synthesized,

D. contacting the separated primer extension products and the specimen with additional first and second primers, resulting in amplification of the specific nucleic acid sequence to form complementary products,

E. separating the primer extension products from the complementary products formed in step D,

F. contacting the first primer extension product separated in step E with a detectably labeled oligonucleotide probe which is complementary thereto to form a product of the labeled probe and first primer extension product, and

G. detecting the complementary product formed in step F as an indication of the presence of the predetermined nucleic acid in the specimen,

provided that prior to detection step G, the specific binding ligand of the first primer is complexed with a receptor therefor which is bound to a solid support material.

2. A method for the detection of a specific nucleic acid sequence of a predetermined nucleic acid, the sequence having two complementary strands, the method comprising:

A. contacting a specimen suspected of containing a predetermined nucleic acid with

(1) first and second oligonucleotide primers complementary to the strands of the specific nucleic acid sequence, at least the first primer being labeled with a specific binding ligand, and

(2) a solid support material having a receptor for the specific binding ligand bound thereto,

so as to form a mixture of hybridized products of the first and second primers and the complementary strands, at least the first primer being bound to the support material through a complex of the specific binding ligand and the receptor,

B. forming first and second extension products of the primers in the hybridized products, which extension products, when separated from their complements, can serve as templates for synthesis of the extension products of the primers,

C. separating the primer extension products from the templates on which they were synthesized, the first primer extension product being insoluble,

D. contacting the separated primer extension products and the specimen with additional first and second primers, resulting in amplification of the specific nucleic acid sequence to form complementary products,

E. separating the primer extension products from the complementary products formed in step D,

F. contacting the insoluble first primer extension product separated in step E with a detectably labeled oligonucleotide probe which is complementary thereto to form a product of the labeled probe and insoluble first primer extension product, and

G. detecting the insoluble complementary product formed in step F as an indication of the presence of the predetermined nucleic acid in the specimen.

3. A method for the detection of a specific nucleic acid sequence of a predetermined nucleic acid, the sequence having two complementary strands, the method comprising:

A. contacting a specimen suspected of containing the predetermined nucleic acid with first and second oligonucleotide primers complementary to the specific nucleic acid sequence strands, at least the first primer being labeled with a specific binding ligand, so as to form a mixture of hybridized products of the first and second primers and the complementary strands,

B. forming first and second extension products of the primers in the hybridized products, which extension products, when separated from their complements, can serve as templates for synthesis of the extension products of the primers,

C. separating the primer extension products from the templates on which they were synthesized,

D. contacting the separated primer extension products and the specimen with additional first and second primers, resulting in amplification of the specific nucleic acid sequence to form complementary products,

E. separating the primer extension products from the complementary products formed in step D,

F. contacting the first primer extension product separated in step E with a detectably labeled oligonucleotide probe which is complementary thereto to form a product of the labeled probe and first primer extension product,

G. contacting the complementary product formed in step F with a solid support material to which is bound a receptor for the specific binding ligand of the first primer, thereby insolubilizing the probe-first primer complementary product through complexation of the specific binding ligand and receptor therefor, and

H. detecting the insolubilized complementary product formed in step G as an indication of the presence of the predetermined nucleic acid in the specimen.

4. The method as claimed in any of claims 1 to 3 wherein the solid support material is a polymeric particle.

5. The method as claimed in claim 4 wherein the particle is composed of polymers formed from ethylenically unsaturated polymerizable monomers having carboxy, activated 2-chloroethylsulfonyl, active halogen or vinylsulfonyl groups.

6. The method as claimed in any of claims 1 to 5 for the detection of HIV-I.

7. The method as claimed in any of claims 1 to 6 wherein the specific binding moiety is biotin or a derivative thereof, and the receptor is avidin or a derivative thereof.

8. The method as claimed in any of claims 1 to 6 wherein the specific binding moiety is an immunological species.

9. The method as claimed in any of claims 1 to 6 wherein the specific binding moiety is either a lectin or sugar, and the receptor is, respectively, a sugar or a lectin.

10. A method for the detection of a specific nucleic acid sequence in the gag region of HIV-I DNA, the sequence having two complementary strands, the method comprising:

A. contacting a specimen suspected of containing HIV-I DNA with

(1) first and second oligonucleotide primers complementary to the strands of a specific nucleic acid sequence in the gag region of HIV-I DNA, at least the first primer being labeled with biotin, and

(2) polymeric particles having an average particle size of from 0.1 to 10 $\mu$meter and having avidin covalently bound thereto, so as to form a mixture of hybridized products of the first and second primers and HIV-I complementary strands, the first primer being bound to the particles through a biotin-avidin complex,

B. forming first and second extension products of the primers in the hybridized products, which extension products, when separated from their complements, can serve as templates for synthesis of the extension products of the primers,

15

C. separating the primer extension products from the templates on which they were synthesized, the first primer extension product being insoluble,

D. contacting the separated primer extension products and the specimen with additional first and second primers, resulting in amplification of the specific HIV-I nucleic acid sequence to form complementary products,

E. separating the primer extension products from the complementary products formed in step D,

F. contacting the insoluble first primer extension product separated in step E with an enzyme-labeled oligonucleotide probe which is complementary thereto to form a product of the labeled probe and insoluble first primer extension product, and

G. detecting the insoluble complementary product formed in step F by contacting it with a composition which provides a dye in the presence of the enzyme as an indication of the presence of HIV-I in the specimen.

11. A method for the detection of a specific nucleic acid sequence in HLA DNA, the sequence having two complementary strands, the method comprising:

A. contacting a specimen suspected of containing HLA DNA with

(1) first and second oligonucleotide primers complementary to the strands of a specific nucleic acid sequence in HLA DNA, at least the first primer being labeled with biotin, and

(2) polymeric particles having an average particle size of from 0.1 to 10 μmeter and having avidin covalently bound thereto,

so as to form a mixture of hybridized products of the first and second primers and HLA DNA complementary strands, the first primer being bound to the particles through a biotin avidin complex,

B. forming first and second extension products of the primers in the hybridized products, which extension products, when separated from their complements, can serve as templates for synthesis of the extension products of the primers,

C. separating the primer extension products from the templates on which they were synthesized, the first primer extension product being insoluble,

D. contacting the separated primer extension products and the specimen with additional first and second primers, resulting in amplification of the specific HLA DNA nucleic acid sequence to form complementary products,

E. separating the primer extension products from the complementary products formed in step D,

F. contacting the insoluble first primer extension product separated in step E with an enzyme-labeled oligonucleotide probe which is complementary thereto to form a product of the labeled probe and insoluble first primer extension product, and

G. detecting the insoluble complementary product formed in step F by contacting it with a composition which provides a dye in the presence of the enzyme as an indication of the presence of HLA DNA in the specimen.

12. A method for the detection of a specific nucleic acid sequence in human β-globin DNA, the sequence having two complementary strands, the method comprising:

A. contacting a specimen suspected of containing human β-globin DNA with

(1) first and second oligonucleotide primers complementary to the strands of a specific nucleic acid sequence in human β-globin DNA, at least the first primer being labeled with biotin, and

(2) polymeric particles having an average particle size of from 0.1 to 10 μmeter and having avidin covalently bound thereto,

so as to form a mixture of hybridized products of the first and second primers and the human β-globin DNA complementary strands, the first primer bound to the particles through a biotin-avidin complex,

B. forming first and second extension products of the primers in the hybridized products, which extension products, when separated from their complements, can serve as templates for synthesis of the extension products of the primers,

C. separating the primer extension products from the templates on which they were synthesized, the first primer extension product being insoluble,

D. contacting the separated primer extension products and the specimen with additional first and second primers, resulting in amplification of the specific human β-globin DNA nucleic acid sequence to form complementary products,

E. separating the primer extension products from the complementary products formed in step D,

F. contacting the insoluble first primer extension product separated in step E with an enzyme-labeled oligonucleotide probe which is complementary thereto to form a product of the labeled probe and insoluble first primer extension product, and

G. detecting the insoluble complementary product formed in step F by contacting it with a composition which provides a dye in the presence of the enzyme as an indication of the presence of human β-globin DNA in the specimen.

13. A method for the detection of a specific nucleic acid sequence in the gag region of HIV-I DNA, the sequence having two complementary strands, the method comprising:

A. contacting a specimen suspected of containing HIV-I DNA with first and second oligonucleotide primers complementary to the strands of a specific nucleic acid sequence in the gag region of HIV-I DNA, at least the first primer being labeled with biotin,

so as to form a mixture of hybridized products of the first and second primers and the complementary strands,

B. forming first and second extension products of the primers in the hybridized products, which extension products, when separated from their complements, can serve as templates for synthesis of the extension products of the primers,

C. separating the primer extension products from the templates on which they were synthesized,

D. contacting the separated primer extension products and the specimen with additional first and second primers, resulting in amplification of the specific HIV-I DNA nucleic acid sequence to form complementary products,

E. separating the primer extension products from the complementary products formed in step D,

F. contacting the first primer extension product separated in step E with an enzyme-labeled oligonucleotide probe which is complementary thereto to form a product of the labeled probe and first primer extension product,

G. contacting the complementary product formed in step F with polymeric particles having an average particle size of from 0.1 to 10 μmeters, and having avidin covalently bound thereto, thereby insolubilizing the probe-first primer complementary product through complexation of biotin and avidin, and

H. detecting the insolubilized complementary product formed in step G by contacting it with a composition which will provide a dye in the presence of the enzyme as an indication of the presence of HIV-I in the specimen.

14. A method for the detection of a specific nucleic acid sequence in HLA DNA, the sequence having two complementary strands, the method comprising:

A. contacting a specimen suspected of containing HLA DNA with first and second oligonucleotide primers complementary to the strands of a specific HLA DNA nucleic acid sequence, at least the first primer being labeled with biotin,

so as to form a mixture of hybridized products of the first and second primers and the complementary strands,

B. forming first and second extension products of the primers in the hybridized products, which extension products, when separated from their complements, can serve as templates for synthesis of the extension products of the primers,

C. separating the primer extension products from the templates on which they were synthesized,

D. contacting the separated primer extension products and the specimen with additional first and second primers, resulting in amplification of the specific HLA DNA nucleic acid sequence to form complementary products,

E. separating the primer extension products from the complementary products formed in step D,

F. contacting the first primer extension product separated in step E with an enzyme-labeled oligonucleotide probe which is complementary thereto to form a product of the labeled probe and first primer extension product,

G. contacting the complementary product formed in step F with polymeric particles having an average particle size of from 0.1 to 10 μmeters, and having avidin covalently bound thereto, thereby insolubilizing the probe-first primer complementary product through complexation of biotin and avidin, and

H. detecting the insolubilized complementary product formed in step G by contacting it with a composition which provides a dye in the presence of the enzyme as an indication of the presence of HLA DNA in the specimen.

15. A method for the detection of a specific nucleic acid sequence in human β-globin DNA, the sequence having two complementary strands, the method comprising:

A. contacting a specimen suspected of containing human β-globin DNA with first and second oligonucleotide primers complementary to the strands of the specific nucleic acid sequence in human β-globin DNA, at least the first primer being labeled with biotin,

so as to form a mixture of hybridized products of the first and second primers and the complementary strands,

B. forming first and second extension products of the primers in the hybridized products, which

17

extension products, when separated from their complements, can serve as templates for synthesis of the extension products of the primers,

C. separating the primer extension products from the templates on which they were synthesized,

D. contacting the separated primer extension products and the specimen with additional first and second primers, resulting in amplification of the specific human β-globin DNA nucleic acid sequence to form complementary products,

E. separating the primer extension products from the complementary products formed in step D,

F. contacting the first primer extension product separated in step E with an enzyme-labeled oligonucleotide probe which is complementary thereto to form a product of the labeled probe and first primer extension product,

G. contacting the complementary product formed in step F with polymeric particles having an average particle size of from 0.1 to 10 μmeters, and having avidin covalently bound thereto, thereby insolubilizing the probe-first primer complementary product through complexation of biotin and avidin, and

H. detecting the insolubilized complementary product formed in step G by contacting it with a composition which provides a dye in the presence of the enzyme as an indication of the presence of human β-globin DNA in the specimen.

16. An aqueous suspension of polymeric particles to which are bound an oligonucleotide primer, the suspension characterized wherein the primer is attached to the particles through a specific binding complex of a specific binding ligand and its receptor.

17. The suspension as claimed in claim 16 wherein the primer is attached to the particles through an avidin-biotin, antibody-antibody, antibody-antigen or sugar-lectin complex.

18. The suspension as claimed in either of claims 16 and 17 wherein the particles are composed of polymers formed from ethylenically unsaturated polymerizable monomers having carboxy, activated 2-chloroethylsulfonyl, active halogen or vinylsulfonyl groups.

19. A diagnostic test kit comprising:
a) first and second oligonucleotide primers complementary to the separate strands of a predetermined nucleic acid sequence,
b) a receptor for the specific binding ligand,
c) a polymerization agent for primer extension, and
d) the deoxyribonucleotide triphosphates dATP, dCTP, dGTP and dTTP,
the kit characterized wherein at least one of the primers is labeled with a specific binding ligand, and the receptor is bound to a solid support material, the receptor provided in the kit either in separate packaging or as complexed with the specific binding ligand on one of the primers.